(19) Europäisches Patentamt
European Patent Office
Office européen des brevets

(11) Veröffentlichungsnummer : **0 183 077**
**B1**

# (12) EUROPÄISCHE PATENTSCHRIFT

(45) Veröffentlichungstag der Patentschrift : **26.04.89**

(51) Int. Cl.⁴ : **A 61 K 7/08**, A 61 K 7/06

(21) Anmeldenummer : **85113845.3**

(22) Anmeldetag : **31.10.85**

## (54) Haarkonditionierungsmittel mit Perlglanz.

(30) Priorität : **09.11.84 DE 3440935**

(43) Veröffentlichungstag der Anmeldung :
**04.06.86 Patentblatt 86/23**

(45) Bekanntmachung des Hinweises auf die Patenterteilung : **26.04.89 Patentblatt 89/17**

(84) Benannte Vertragsstaaten :
**AT BE CH DE FR GB IT LI LU NL SE**

(56) Entgegenhaltungen :
**EP-A- 0 130 609**
**EP-A- 0 141 593**
**WO-A-83 /023 90**
**FR-A- 1 163 386**
**PATENTS ABSTRACTS OF JAPAN, Band 7, Nr. 46 (C-153) [1191] 23. Februar 1983; & JP - A - 57 200 308 (RAION K.K.) 08.12.1982**

(73) Patentinhaber : **Wella Aktiengesellschaft**
**Berliner Allee 65**
**D-6100 Darmstadt (DE)**

(72) Erfinder : **Lang, Günther, Dr.**
**Auf der Roten Erde 10 B**
**D-6107 Reinheim 5 (DE)**
Erfinder : **Gross, Paul**
**Forstweg 54**
**D-6100 Darmstadt (DE)**
Erfinder : **Schröder, Friedel**
**Viktoriaplatz 1**
**D-6100 Darmstadt (DE)**
Erfinder : **Grundmann, Karin**
**Ringstrasse 48**
**D-6102 Pfungstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 183 077 B1**

Jouve, 18, rue St-Denis, 75001 Paris, France

**Beschreibung**

Durch öfteres Bleichen, Dauerwellen und Färben, aber auch durch häufiges Waschen der Haare mit entfettenden Tensiden sowie durch intensive Einwirkung von Sonnenlicht kommt es zu einer Schädigung der Haarstruktur. Das Haar wird spröde, und es verliert seinen Glanz. Weiterhin lädt sich das Haar beim Kämmen elektrostatisch auf, und die aufgerauhte Haaroberfläche verursacht Verfilzungen sowie Verknotungen des Haares. Hierdurch wird das Kämmen sehr erschwert. Haarkonditionierungsmittel mit einer kämmbarkeitsverbessernden und pflegenden Wirkung haben daher eine erhebliche Bedeutung erlangt. Durch die Anwendung von Haarkonditionierungsmitteln, wie z. B. Haarkuren und Rinses, soll insbesondere die Kämmbarkeit des Haares verbessert und die elektrostatische Aufladung beim Kämmen verhindert werden. Die Haarkonditionierungsmittel liegen vorwiegend in Form von Öl-in-Wasser-Emulsionen, beispielsweise als sogenannte Creme-Rinses, vor und enthalten als Gerüstbestandteile halbfeste oder feste Substanzen, wie beispielsweise Paraffinöl, Vaseline, Wollfett, Wollfettalkohole, Fettsäureester und Kohlenwasserstoffwachse. Als Emulgatoren für solche Emulsionen werden normalerweise quaternäre Ammoniumverbindungen wie oxethylierte Alkylammoniumphosphate, Alkyltrimethylammoniumchloride, Dialkyldimethylammoniumchloride, Alkyldimethylbenzylammoniumchloride und Alkylpyridiniumchloride, allein oder in Kombination mit nichtionogenen Emulgatoren, verwendet.

Die am häufigsten angewandten Rezepturen enthalten insbesondere quaternäre Ammoniumsalze und Fettalkohole. So werden beispielsweise in dem Kurzreferat der JP-A 57-200 308, veröffentlicht in Patents Abstracts of Japan, Bd. 7, Nr. 46 (C-153) (1191), Haarpflegemittel beschrieben, die eine Mischung aus bestimmten quaternären Ammoniumsalze, $C_{12}$ bis $C_{14}$-Fettalkohole, Vaseline sowie bestimmten Kondensationsprodukten enthalten. Derartige Zusammensetzungen besitzen jedoch keinen Perlglanz sondern in der Regel ein milchigtrübes Aussehen.

Ebenfalls sind aus der FR-A 1 163 386 bakterizid wirkende Shampoozusammensetzungen bekannt, die eine quaternäre Ammoniumverbindung, zum Beispiel Cetyl- oder Lauryltrimethylammoniumbromid und Cetyl- oder Laurylpyridiniumbromid, sowie bestimmte Fettsäureethanolamide, beispielsweise Palmkern, Ölsäure-, Palmitinsäure-, Stearinsäure- und Rizinolsäuremono- oder diethanolamid, enthalten. Gegebenenfalls sollen diese Shampoozusammensetzungen zusätzlich Cetylalkohol enthalten können. Diese Shampoozusammensetzungen weisen jedoch keinen Perglanz auf.

Obwohl es in der Vergangenheit nicht an Versuchen gefehlt hat, Haarkonditionierungsmitteln ein perlglänzendes, ästhetischeres Aussehen zu verleihen, ist es bisher nicht gelungen, ein solches Produkt mit echtem Perlglanz zur Verfügung zu stellen. Während perglänzende Rezepturen auf dem Gebiet der Shampoos Stand der Technik sind und häufig angewandt werden, ist es offensichtlich problematisch, stabile kationische, fettalkoholhaltige Mittel mit Perlglanz herzustellen. So ist zum Beispiel in der US-A 4 275 055 eine perlglänzende, kationische Haarkur beschrieben, die jedoch einen hohen Anteil an hochschmelzendem, schwerlöslichem Stearyldimethylbenzylammoniumchlorid und keinen Fettalkohol enthält. Andere Haarkonditionierungsmittel mit Perlglanz sind nicht rein kationisch, sondern enthalten hohe Anteile an anionischen und nichtionischen Tensiden. Solche bekannten perlglänzenden Haarkonditionierungsmittel mit zufriedenstellenden Eigenschaften enthalten zudem verhältnismäßig teure Rohstoffe.

Aufgabe der vorliegenden Erfindung war es daher, perlglänzende Haarkonditionierungsmittel mit guten Gebrauchseigenschaften auf der Basis der relativ preiswerten Rohstoffe Fettalkohol und quaternäre Verbindung zur Verfügung zu stellen.

Hierzu wurde nun gefunden, daß diese Aufgabe durch Haarkonditionierungsmittel mit Perlglanz auf der Basis einer quaternären Verbindung und eines Fettalkohols, bestehend aus

A) 0,2 bis 10 Gew.% Kokosfettsäuremonoethanolamid,

B) 0,4 bis 10 Gew.% eines geradkettigen Fettalkohols mit m Kohlenstoffatomen, wobei m eine Zahl von 14 bis 18 darstellt, oder eines Gemisches dieser Fettalkohole,

C) 0,1 bis 4 Gew.% einer quaternären Verbindung der allgemeinen Formel

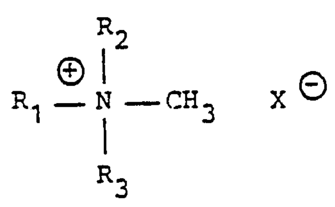

und/oder

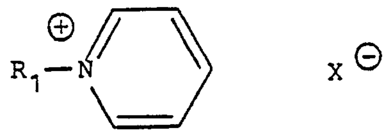

wobei $R_1$ die Bedeutung Alkylrest mit n Kohlenstoffatomen (n = 12 bis 22), Kokosamidoethyl, Kokosamidopropyl, R'—Y— mit R' = $C_{12}$ bis $C_{20}$-Alkyl und

$$Y = -O-\overset{\overset{O}{\|}}{C}-CH_2- \text{ oder}$$

$$-\overset{\overset{O}{\|}}{C}-O-CH_2-CH_2-$$

hat, $R_2$ einen der Reste $CH_3$, $CH_2CH_2OH$ oder $CH_2CH_3$ darstellt, $R_3CH_3$, $CH_2CH_2OH$, Benzyl oder Acetamidyl bedeutet und $X^{\ominus} = Cl^-$, $Br^-$, $CH_3SO_4^-$, 1/2 $SO_4^{2-}$, Lactat⁻ oder Acetat⁻ ist,

D) 70 bis 99,3 Gew.% Wasser und

E) 0 bis 24 Gew.% üblichen kosmetischen Zusatzstoffen,

wobei die Summe, gebildet aus der Anzahl der Kohlenstoffatome des Fettalkohols (m) und der Anzahl der Kohlenstoffatome des Restes $R_1$ der quaternären Verbindung (n) größer oder gleich 29 ist ($m + n \geq 29$), unter der Voraussetzung, daß das Gewichtsverhältnis der Komponente A zur Komponente B zwischen 0,1 bis 3 liegt und das Gewichtsverhältnis der Komponente C zu der Summe der Komponenten A und B zwischen 0,05 und 0,5 beträgt, sowie unter der weiteren Voraussetzung, daß der Gewichtsanteil der kosmetischen Zusatzstoffe nicht größer ist als der Gewichtsanteil aus der Summe der Komponenten A, B und C, gelöst wird.

Die erfindungsgemäßen Haarkonditionierungsmittel weisen eine sehr gute kämmbarkeitsverbessernde Wirkung, eine gleichbleibende Viskosität und ein stabiles perlglänzendes Aussehen auf.

Besonders vorteilhaft ist es, wenn die Komponente A (Kokosfettsäuremonoethanolamid) in einer Menge von 0,5 bis 4 Gew.% in den Mitteln enthalten ist.

Die besonders bevorzugte Einsatzmenge für die Komponente B (Fettalkohol) beträgt 0,8 bis 5 Gew.%. Als Fettalkohole kommen alle geradkettigen Fettalkohole mit 14 bis 18 Kohlenstoffatomen, wie zum Beispiel Tetradecanol, Cetylalkohol und Stearylalkohol, in Betracht. Die Komponente B kann ferner aus einem Fettalkoholgemisch, beispielsweise aus einem 50 : 50 Gemisch aus Cetylalkohol und Stearylalkohol (Handelsprodukt LANETTE® oder Firma Henkel, Düsseldorf), bestehen ; die durchschnittliche Anzahl der Kohlenstoffatome der Fettalkoholkomponente B beträgt dann m = 17.

Haarkonditionierungsmittel mit besonders schönem und intensivem Perlglanz werden erhalten, wenn die Komponente B aus Cetylalkohol oder Stearylalkohol oder aus einem Gemisch der beiden Alkohole besteht und die Komonente C aus Cetyltrimethylammoniumchlorid oder Stearyltrimethylammoniumchlorid oder einem Gemisch dieser kationischen Verbindungen besteht. Diese Haarkonditionierungsmittel haben darüber hinaus den Vorteil, daß sie auch bei Temperaturen von über 40 °C einen stabilen Perlglanz aufweisen.

Eine besonders bevorzugte Ausführungsform der beschriebenen Haarkonditionierungsmittel liegt ferner dann vor, wenn das Gewichtsverhältnis der Komponente A zur Komponente B 0,3 bis 2 beträgt.

Als geeignete quaternäre Verbindungen der Komponente C sind vor allem quaternäre Ammoniumverbindungen, wie z. B. Lauryltrimethylammoniumchlorid, Tetradecyltrimethylammoniumchlorid, Cetyltrimethylammoniumchlorid, Stearyltrimethylammoniumchlorid, Cetyldimethylhydroxyethylammoniumdihydrogenphosphat, Cetyldimethylbenzylammoniumchlorid, ferner Pyridiniumsalze, wie z. B. Cetylpyridiniumchlorid, sowie weiterhin andere quaternäre Verbindungen, wie beispielsweise Laurylsäurecholinester-chlorid, Betaincetylester-chlorid und Kokosfettsäureamidopropyldimethylacetamidylammoniumchlorid enthalten, vorzugsweise in einer Menge von 0,1 bis 1,5 Gew.%.

Die Hauptmenge der erfindungsgemäßen Mittel besteht aus Wasser, wobei der Wassergehalt vorzugsweise 90 bis 97 Gew.% beträgt. Obwohl Leitungswasser verwendet werden kann, wenn es ein relativ geringes Maß an Ionen enthält, ist es bevorzugt, entionisiertes Wasser zu verwenden.

Die hier beschriebenen Haarkonditionierungsmittel können darüberhinaus alle diejenigen kosmetischen Zusatzstoffe enthalten, die üblicherweise in Haarkonditionierungsmitteln eingesetzt werden, insbesondere Farbstoffe, Parfümöle, Vitamine, Konservierungsmittel, Antioxidantien sowie Wirkstoffe gegen Kopfschuppen. Die vorstehend genannten Zusatzstoffe können jeweils bis zu einer Menge von 1

Gew.% enthalten sein. Weitere übliche Zusatzstoffe sind z. B. kosmetisch wirksame Öle, wie z. B. Avocadoöl, oder Pflegestoffe wie z. B. Lanolin, Cholesterin und Pantothensäure, wobei diese Zusatzstoffe in einer Menge von bis zu 10 Gew.% enthalten sein können. Die Mittel können als Zusatzstoffe ferner kationische Harze sowie Verdicker, z. B. Stärke, Cellulosederivate oder hochdisperse Kieselsäure (z. B. Aerosil ®300 der Firma Degussa, Hanau, BRD) in einer Menge bis zu 2 Gew.% enthalten.

Die erfindungsgemäßen Haarkonditionierungsmittel können auch in Form von Aerosolpräparaten vorliegen und enthalten dann als weiteren üblichen Zusatzstoff bis zu 10 Gew.% Treibmittel. Als Treibmittel können Chlorfluoralkane, wie z. B. $CCl_3F$, $CCl_2F_2$, $C_2Cl_3F_3$, $CCl_2F—CCL_2F$, $CHCl_2F$, $CHClF_2$ und $CClF_2—CClF_2$, leichtflüchtige Kohlenwasserstoffe, wie z. B. n-butan und n-Propan, oder auch Dimethylether, $CO_2$, $N_2O$, $N_2$, $CH_2Cl_2$ und $CCl_3—CH_3$ Verwendung finden.

Die Herstellung der erfindungsgemäßen Mittel erfolgt nach den für kosmetische Emulsionen üblichen Herstellungsverfahren, indem zum Beispiel die hydrophoben Bestandteile (Komponenten A und B sowie gegebenenfalls enthaltene hydrophobe Zusatzstoffe) zunächst bei 90 °C geschmolzen werden, und sodann die hydrophilen Bestandteile (Komponente C sowie hydrophobe Zusatzstoffe) in Wasser gelöst werden. Anschließend wird die wäßrige Lösung auf 90 °C erwärmt und die Schmelze der hydrophoben Bestandteile unter Rühren einemulgiert. Schließlich wird die entstandene Emulsion abgekühlt.

Die nachstehenden Beispiele sollen den Gegenstand der Erfindung näher erläutern.

## Beispiele 1 bis 20

Zur Darstellung des Auftretens von Perlglanz bei den Mitteln in Abhängigkeit von der Alkylkettenlänge des verwendeten Fettalkohols und des Alkylrestes an der quaternären Verbindung wurden bei einem erfindungsgemäßen Haarkonditionierungsmittel der Zusammensetzung

| | | |
|---|---|---|
| A) | 1,35 Gew.% | Kokosfettsäuremonoethanolamid |
| B) | 2,00 Gew.% | Fettalkohol |
| C) | 0,55 Gew.% | quaternäre Verbindung |
| | 0,30 Gew.% | Parfümöl |
| | 0,10 Gew.% | Farbstoff |
| | 95,70 Gew.% | Wasser, entsalzt |
| | 100,00 Gew.% | |

Fettalkohole mit Alkylkettenlängen von $C_{12}$ bis $C_{18}$ mit Alkyltrimethylammoniumsalzen der Kettenlängen $C_{12}$ bis $C_{18}$ kombiniert.

Das Ergebnis der Versuche ist nachfolgend in Form der Tabelle 1 dargestellt. Wie aus der Tabelle 1 zu ersehen ist, ergibt die oben genannte Zusammensetzung nur für solche Kombinationen von Fettalkohol und quaternärer Verbindung Perlglanz, in denen die Summe der Kohlenstoffatome des Alkylrestes des Fettalkohols und der quaternären Verbindung größer oder gleich 29 ist (Beispiele 4, 5, 8-10, 12-15 und 17-20).

(Siehe Tabelle 1 Seite 5 f.)

Tabelle 1 Auftreten von Perlglanz in Abhängigkeit von der Alkylkettenlänge des Fettalkohols ($C_m$) und des Alkylrestes der quaternären Verbindung ($C_n$)

| Fettalkohol ($C_m$) / quaternäre Verbindung ($C_n$) | $C_{12}$ Dodecylalkohol | $C_{14}$ Tetradecylalkohol | $C_{16}$ Cetylalkohol | $C_{16/18}$ Cetylstearylalkohol | $C_{18}$ Stearylalkohol |
|---|---|---|---|---|---|
| $C_{12}$ Lauryltrimethylammoniumchlorid | 1 – | 2 – | 3 – | 4 + | 5 + |
| $C_{14}$ Tetradecyltrimethylammoniumchlorid | 6 – | 7 – | 8 + | 9 + | 10 + |
| $C_{16}$ Cetyltrimethylammoniumchlorid | 11 – | 12 + | 13 + | 14 + | 15 + |
| $C_{18}$ Stearyltrimethylammoniumchlorid | 16 – | 17 + | 18 + | 19 + | 20 + |

\+ = Perlglanz
— = kein Perlglanz
breit umrandet = erfindungsgemäße Zusammensetzungen ($C_m + C_n \geq 29$)

Beispiele 21 bis 25 Haarkonditionierungsmittel

Die Beispiele 21-25 zeigen die Verwendbarkeit weiterer kationischer Verbindungen. Jeweils 20 g der nachstehenden Haarkonditionierungsmittel werden im gewaschenen Haar verteilt und nach einer Einwirkungszeit von 3 bis 5 Minuten mit Wasser ausgespült. Das Haar hat eine gute Kämmbarkeit und einen ausgezeichneten Griff erhalten. Die Haarkonditionierungsmittel zeigen auch nach längerer Lagerung einen stabilen Perlglanz. (Angaben in Gewichtsprozent).

| Beispiel Nr.: | | 21 | 22 | 23 | 24 | 25 |
|---|---|---|---|---|---|---|
| A) | Kokosfettsäuremonoethanolamid | 1,36 | 1,76 | 1,76 | 1,76 | 1,76 |
| B) | Stearylalkohol | 1,90 | 1,90 | 1,90 | 1,90 | 1,90 |
| C) | Cetyldimethylbenzylammoniumchlorid | 0,54 | - | - | - | - |
| | Cetylpyridiniumchlorid | - | 0,53 | - | - | - |
| | $C_{20-22}$-Alkyltrimethylammoniumchlorid | - | - | 0,66 | - | - |
| | Cetyldimethylhydroxyethylammoniumdihydrogenphosphat | - | - | - | 0,54 | - |
| | Kokosfettsäureamidopropyldimethylacetamidylammoniumchlorid | - | - | - | - | 1,27 |
| | Parfümöl | 0,30 | 0,30 | 0,30 | 0,30 | 0,70 |
| | Farbstoff | 0,10 | 0,10 | 0,10 | 0,10 | 0,10 |
| | Wasser | 95,80 | 95,41 | 95,28 | 95,40 | 94,27 |
| | | 100,00 | 100,00 | 100,00 | 100,00 | 100,00 |
| | A/B | 0,70 | 0,92 | 0,92 | 0,92 | 0,92 |
| | $\frac{C}{A+B}$ | 0,16 | 0,15 | 0,22 | 0,15 | 0,34 |

Beispiele 26 bis 30 Haarkonditionierungsmittel (Angaben in Gew.%)

| Beispiel Nr.: | 26 | 27 | 28 | 29 | 30 |
|---|---|---|---|---|---|
| A) Kokosfett-säuremono-ethanolamid | 0,76 | 1,19 | 1,27 | 1,46 | 2,20 |
| B) Stearylalko-hol | 2,54 | 2,39 | 1,81 | 1,69 | 1,10 |
| C) Stearyltrime-thylammonium-chlorid | 0,65 | 0,40 | 0,86 | 0,73 | 0,65 |
| Parfümöl | 0,20 | 0,20 | 0,20 | 0,20 | 0,20 |
| Wasser | 95,85 | 95,82 | 95,86 | 95,92 | 95,85 |
| | 100,00 | 100,00 | 100,00 | 100,00 | 100,00 |
| A /B | 0,30 | 0,50 | 0,70 | 0,86 | 2,00 |
| C / A+B | 0,19 | 0,11 | 0,28 | 0,27 | 0,19 |

Beispiele 31 und 32 mittelviskoses Haarkonditionierungsmittel

Die nachstehenden Beispiele 31 und 32 stellen stabile, perlglänzende Haarkonditionierungsmittel mittlerer Viskosität mit guten anwendungstechnischen Eigenschaften dar. Sie zeigen die Verwendbarkeit von leicht abbaubaren und daher die Umwelt besonders wenig belastenden kationischen Tensiden als quaternäre Verbindung für die erfindungsgemäßen Mittel. (Angaben in Gew.%)

| Beispiel Nr. | 31 | 32 |
|---|---|---|
| A) Kokosfettsäuremonoethanol-amid | 1,76 | 1,76 |
| B) Cetylstearylalkohol | 1,96 | 1,96 |
| C) Betaincetylester-chlorid | 0,60 | - |
| C) Laurylsäurecholinester-chlorid | - | 0,60 |
| Parfümöl | 0,50 | 0,50 |
| Farbstoff | 0,10 | 0,10 |
| Wasser | 95,08 | 95,08 |
| | 100,00 | 100,00 |
| A/B | 0,90 | 0,90 |
| C / A+B | 0,16 | 0,16 |

Beispiel 33 hochviskoses Haarkonditionierungsmittel

Das nachstehend angeführte Haarkonditionierungsmittel stellt eine hochviskose, perlglänzende Haarkur dar, welche wie für die Beispiele 21 bis 25 beschrieben angewendet wird. Geschädigtes Haar zeigt nach der Anwendung eine gute Kämmbarkeit sowie eine größere Fülle (Body-Effekt).

| | |
|---|---|
| A) Kokosfettsäuremonoethanolamid | 3,10 Gew.% |
| B) Cetylstearylalkohol | 4,42 Gew.% |
| C) Cetyltrimethylammoniumchlorid | 1,24 Gew.% |
| Hochdisperse Kielselsäure (Aerosil ®300) | 0,50 Gew.% |
| Parfümöl | 0,50 Gew.% |
| Wasser | 90,24 Gew.% |
| | 100,00 Gew.% |

A/B = 0,7
C/(A + B) = 0,16

Beispiel 34 Aerosol-Schaumkur

Bei dem nachstehend angeführten Mittel ist, falls es in einem durchsichtigen Aerosolbehälter abgefüllt wird, schöner Perlglanz zu beobachten. Der Aerosolbehälter wird vor dem Austragen des Produktes geschüttelt. Das Mittel tritt in Form eines sahnigen Schaumes mit sehr guter konditionierender Wirkung aus. Die Anwendung des Mittels erfolgt in der bei den Beispielen 21 bis 25 beschriebenen Weise.

| | |
|---|---|
| A) Kokosfettsäuremonoethanolamid | 0,62 Gew.% |
| B) Cetylstearylalkohol | 0,88 Gew.% |
| C) Cetyltrimethylammoniumchlorid | 0,13 Gew.% |
| Farbstoff | 0,30 Gew.% |
| Parfümöl | 0,20 Gew.% |
| Wasser | 97,87 Gew.% |
| | 100,00 Gew.% |

92 Gew.% der obigen Zusammensetzung werden gemeinsam mit 8 Gew.% eines Treibmittelgemisches, bestehend aus 50 Gew.% Propan und 50 Gew.% Butan, in einen durchsichtigen Aerosolbehälter abgefüllt.

## Patentansprüche

1. Haarkonditionierungsmittel mit Perlglanz auf der Basis einer quaternären Verbindung und eines Fettalkohols, bestehend aus

A) 0,2 bis 10 Gew.% Kokosfettsäuremonoethanolamid,

B) 0,4 bis 10 Gew.% eines geradkettigen Fettalkohols mit m Kohlenstoffatomen, wobei m eine Zahl von 14 bis 18 darstellt, oder eines Gemisches dieser Fettalkohole,

C) 0,1 bis 4 Gew.% einer quaternären Verbindung der allgemeinen Formel

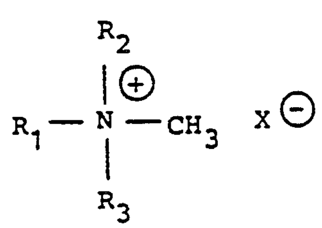

und/oder

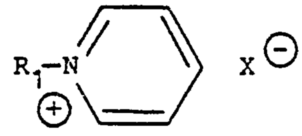

wobei $R_1$ die Bedeutung Alkylrest mit n Kohlenstoffatomen (n = 12 bis 22), Kokosamidoethyl, Kokosamidopropyl, R'—Y— mit R' = $C_{12}$ bis $C_{20}$-Alkyl und

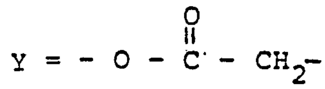

oder

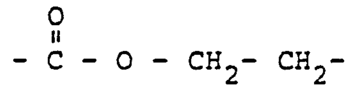

hat,

$R_2$ einen der Reste $CH_3$, $CH_2CH_2OH$ oder $CH_2CH_3$ darstellt,

$R_3CH_3$, $CH_2CH_2OH$, Benzyl oder Acetamidyl bedeutet und

$X^{\ominus} = Cl^-$, $Br^-$, $CH_3SO_4^-$, $1/2\ SO_4^{2-}$, Lactat$^-$ oder Acetat$^-$ ist,

D) 70 bis 99,3 Gew.% Wasser und

E) 0 bis 24 Gew.% üblichen kosmetischen Zustazstoffen,

wobei die Summe, gebildet aus der Anzahl der Kohlenstoffatome des Fettalkohols (m) und der Anzahl der Kohlenstoffatome des Restes $R_1$ der quaternären Verbindung (n), größer oder gleich 29 ist ($m + n \geqslant 29$), unter der Voraussetzung, daß das Gewichtsverhältnis der Komponente A zur Komponente B zwischen 0,1 bis 3 liegt und das Gewichtsverhältnis der Komponente C zur der Summe der Komponenten A und B zwischen 0,05 und 0,5 beträgt, sowie unter der weiteren Voraussetzung, daß der Gewichtsanteil der kosmetischen Zusatzstoffe nicht größer ist als der Gewichtsanteil aus der Summe der Komponenten A, B und C.

2. Mittel nach Anspruch 1, dadurch gekennzeichnet, daß die Komponente A in einer Menge von 0,5 bis 4 Gew.% enthalten ist.

3. Mittel nach Anspruch 1 und 2, dadurch gekennzeichnet, daß die Komponente B in einer Menge von 0,8 bis 5 Gew.% enthalten ist.

4. Mittel nach Anspruch 1 bis 3, dadurch gekennzeichnet, daß die Komponente C in einer Menge von 0,1 bis 1,5 Gew.% enthalten ist.

5. Mittel nach Anspruch 1 bis 4, dadurch gekennzeichnet, daß es 90 bis 97 Gew.% Wasser enthält.

6. Mittel nach Anspruch 1 bis 5, dadurch gekennzeichnet, daß die Komponente B aus Cetylalkohol und/oder Stearylalkohol besteht und die Komponente C aus Cetyltrimethylammoniumchlorid und/oder Stearyltrimethylammoniumchlorid besteht.

7. Mittel nach Anspruch 1 bis 6, dadurch gekennzeichnet, daß das Gewichtsverhältnis der Komponente A zur Komponente B 0,3 bis 2 beträgt.

8. Mittel nach Anspruch 1 bis 7, dadurch gekennzeichnet, daß die quaternäre Verbindung ausgewählt is aus Lauryltrimethylammoniumchlorid, Tetradecyltrimethylammoniumchlorid, Cetyltrimethylammoniumchlorid, Stearyltrimethylammoniumchlorid, Cetyldimethylbenzylammoniumchlorid, Cetylpyridiniumchlorid, Cetyldimethylhydroxyethylammoniumdihydrogenphosphat, Laurylsäurecholinester-chlorid, Betaincetylester-chlorid und Kokosfettsäureamidopropyldimethylacetamidylammoniumchlorid.

## Claims

1. Hair conditioning agent with a pearl gloss, based on a quaternary compound and a fatty alcohol, consisting of

A) 0.2 to 10 % by weight coconut acid monoethanol amide

B) 0.4 to 10 % by weight of a straight-chain fatty alcohol with m carbon atoms, m representing a number from 14 to 18, or a mixture of these fatty alcohols

C) 0.1 to 4 % by weight of a quarternary compound to the general formula

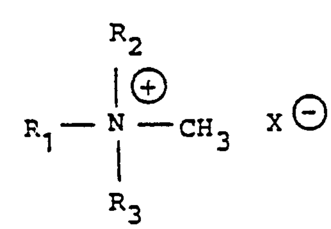

and/or

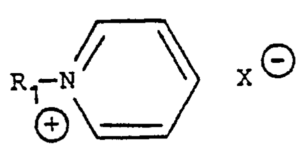

in which $R_1$ denotes an alkyl radical with n carbon atoms (n = 12 to 22), coconut acid amido ethyl, cocinic acid amido propyl, R'—Y— in which R' = $C_{12}$ to $C_{20}$-alkyl and

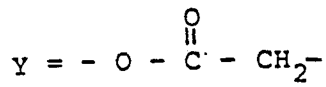

or

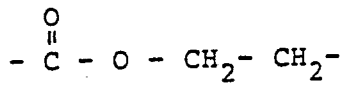

$R_2$ represents one of the radicals $CH_3$, $CH_2CH_2OH$ or $CH_2CH_3$,
$R_3$ = $CH_3$, $CH_2CH_2OH$, benzyl or acetamidyl and
$X^{\ominus}$ = $Cl^-$, $Br^-$, $CH_3SO_4^-$, 1/2 $SO_4^{2-}$, lactate$^-$, or acetate$^-$,
D) 70 to 99.3 % by weight water and
E) 0 to 24 % by weight conventional cosmetic additives,

the sum, constituted by the number of carbon atoms of the fatty alcohol (m) and the number of carbon atoms of the radical $R_1$ of the quaternary compound (n) being greater than or equal to 29 ($m = n \geqslant 29$), subject to the ratio of weights of the component A to the component B being between 0.1 to 3 and the ratio of weight of the component C to the sum of the components A and B being between 0.05 and 0.5, and further subject to the proportion of weight of the cosmetic additives not being greater than the proportion of weight arising from the sum of the components A, B and C.

2. Agent according to Claim 1, characterised in that the component A is contained in a quantity of 0.5 to 4 % by weight.

3. Agent according to Claims 1 and 2, characterised in that the component B is contained in a quantity of 0.8 to 5 % by weight.

4. Agent according to Claims 1 to 3, characterised in that the component C is contained in a quantity of 0.1 to 1.5 % by weight.

5. Agent according to Claims 1 to 4, characterised in that it contains 90 to 97 % by weight of water.

6. Agent according to Claims 1 to 5, characterised in that the component B consists of cetyl alcohol and/or stearyl alcohol, the component C consisting of cetyl trimethyl ammonium chloride and/or stearyl trimethyl ammonium chloride.

7. Agent according to Claims 1 to 6, characterised in that the proportion by weight of component A to component B amounts to 0.3 to 2.

8. Agent according to Claims 1 to 7, characterised in that the quaternary compound is selected from lauryl trimethyl ammonium chloride, tetradecyl trimethyl ammonium chloride, cetyl trimethyl ammonium chloride, stearyl trimethyl ammonium chloride, cetyl dimethyl benzyl ammonium chloride, cetyl pyridinium chloride, cetyl dimethyl hydroxyethyl ammonium dihydrogen phosphate, lauryl cholinic acid ester chloride, betaine cetyl ester chloride and coconut acid amido propyl dimethyl acetamidyl ammonium chloride.

**Revendications**

1. Produit de conditionnement des cheveux à brillant nacré à base d'un composé quaternaire et d'un alcool gras, comprenant

A) 0,2 à 10 % en poids de monoéthanolamide d'acide d'huile de coco,

B) 0,4 à 10 % en poids d'un alcool gras en chaîne linéaire comportant m atomes de carbone, m représentant un nombre de 14 à 18, ou d'un mélange de tels alcools gras,

C) 0,1 à 4 % en poids d'un composé quaternaire répondant à la formule générale :

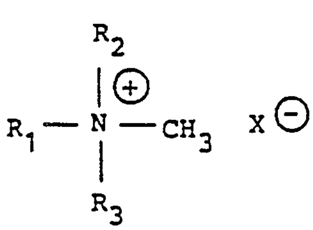

et/ou

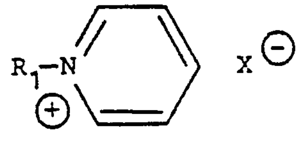

$R_1$ représentant un reste alcoyle comportant n atomes de carbone (n = 12 à 22), un groupe amido-éthyle de coco, un groupe amidopropyle de coco, R'—Y avec R' = alcoyle en $C_{12}$ à $C_{20}$ et

$$Y = -O-\overset{\overset{O}{\|}}{C}-CH_2- \quad \text{ou}$$

$$-\overset{\overset{O}{\|}}{C}-O-CH_2-CH_2-$$

$R_2$ représentant l'un des restes $CH_3$, $CH_2CH_2OH$ ou $CH_2CH_3$,
$R_3$ représentant $CH_3$, $CH_2CH_2OH$, un groupe benzyle ou acétamidyle, et
$X^{\ominus}$ = $Cl^-$, $Br^-$, $CH_3SO_4^-$, 1/2 $SO_4^{2-}$, lactate$^-$ ou acétate$^-$,
D) 70 à 99,3 % en poids d'eau, et
E) 0 à 24 % en poids d'additifs cosmétiques usuels,

la somme du nombre d'atomes de carbone de l'alcool gras (m) et du nombre des atomes de carbone du reste $R_1$ du composé quaternaire (n) étant supérieure ou égale à 29 ($m + n \geqslant 29$), avec la condition que le rapport du poids du constituant A au poids du constituant B soit compris entre 0,1 et 3 et que le rapport du poids du constituant C à la somme des poids des constituants A et B soit compris entre 0,05 et 0,5 et en plus la condition que la proportion en poids des additifs cosmétiques ne soit pas supérieure à la proportion en poids de la somme des constituants A, B et C.

2. Produit selon la revendication 1, caractérisé en ce que le constituant A est contenu dans une proportion de 0,5 à 4 % en poids.

3. Produit selon les revendications 1 et 2, caractérisé en ce que le constituant B est contenu dans une proportion de 0,8 à 5 % en poids.

4. Produit selon les revendications 1 à 3, caractérisé en ce que le constituant C est contenu dans une proportion de 0,1 à 1,5 % en poids.

5. Produit selon les revendications 1 à 4, caractérisé en ce qu'il renferme 90 à 97 % en poids d'eau.

6. Produit selon les revendications 1 à 5, caractérisé en ce que le constituant B est constitué par de l'alcool cétylique et/ou de l'alcool stéarique, et en ce que le constituant C est constitué par du chlorure de cétyltriméthylammonium et/ou du chlorure de stéaryltriméthylammonium.

7. Produit selon les revendications 1 à 6, caractérisé en ce que la proportion en poids du constituant A au constituant B est de 0,3 à 2.

8. Produit selon les revendications 1 à 7, caractérisé en ce que le composé quaternaire est choisi parmi le chlorure de lauryltriméthylammonium, le chlorure de tétradécyltriméthylammonium, le chlorure de cétyltriméthylammonium, le chlorure de stéaryltriméthylammonium, le chlorure de cétyldiméthylbenzylammonium, le chlorure de cétylpyridinium, le monophosphate de cétyldiméthylhydroxyéthylammonium, le chlorure de l'ester cholique de l'acide laurique, le chlorure de l'ester cétylique de la bétaïne et le chlorure d'amidopropyldiméthylacétamidylammonium de l'acide d'huile de coco.